# EUROPEAN PATENT SPECIFICATION

(11) **EP 1 360 969 B1**
(45) Date of publication and mention of the grant of the patent: **22.11.2006**
(21) Application number: 01929667.2
(22) Date of filing: 10.05.2001
(51) Int. Cl.: A61M 5/315

(54) **SYRINGE**
SPRITZE
SERINGUE

(30) Priority: 10.01.2001 ES 200100060
(43) Date of publication of application: 12.11.2003
(73) Proprietor: Page 65, S.L., 48992 Getxo, Vizcaya (ES); SCB, S.A., 48930 Getxo, Vizcaya (ES)
(72) Inventor: Acha Gandarias, Pedro, 48992 Getxo, Vizcaya (ES)
(74) Representative: Primo de Rivera y Urquijo, Jose A.
(86) International application number: PCT/ES2001/000182
(87) International publication number: WO 2002/055141

(56) References cited:
- EP-A1- 0 451 314
- FR-A1- 2 636 537
- US-A- 2 842 128
- US-A- 3 819 091
- US-A- 3 990 446
- US-A- 4 324 241
- US-A- 5 569 210
- US-A- 5 582 595

## Description

### OBJECT OF THE INVENTION

The present invention refers to a syringe and its method of use, which is made up of a working instrument to be employed mainly in the health field for administering medicinal injections and extracting blood or bodily fluids, among other applications. The invention may also be used in other sectors that require syringes to be used for handling fluids, in laboratories, factories of different kinds of activity, or in veterinary applications. The invention also refers to a method for absorbing and expelling a liquid by means of the syringe, which can be used with one hand.

### DESCRIPTION OF THE STATE OF THE ART

In the state of the art many different types of syringes are used, adapted to every need depending on the type of work being carried out. There are different syringes according to the sector and the application for which they are used, but the handling of traditional syringes is common and identical in all fields.

Traditional syringes require to be handled with a greater or lesser degree of stability depending on the work in which they are to be used, since using a syringe to suck up an amount of water from a glass and then distribute it in smaller receptacles does not require the same attention and stability as piercing a vein to extract blood from a person.

The main drawback or problem of traditional syringes is that both hands are needed to use them.

Having both hands occupied is hazardous, since it is possible that an unexpected movement on the part of the patient may cause the needle of the syringe to injure the patient or the syringe user.

To prevent the above-mentioned hazard, there are different syringes or adaptors for traditional syringes which allow them to be used with only one hand.

US Patent Number 3,819,091 describes an adaptor for traditional syringes which is made up of a handle which facilitates the sucking operation of syringes using only one hand. This adaptor resolves the problem of stable sucking with only one hand in syringes, but also increases manufacturing costs as both a syringe and an adaptor have to be manufactured. Due to the fact that the adaptor is only used for sucking and for a specific diameter of syringe, once the syringe is full it is necessary to detach it from the adaptor and use it as a traditional syringe for the purposes of injection or expulsion.

US Patent Number 3,990,446 describes a syringe that allows sucking with only one hand but not the expulsion of the liquid, which must be carried out with both hands. This syringe is longer than traditional ones, since to carry out aspiration it is necessary to have a fixed surface to support the fingers, which is situated at a determinate distance along which the side supports situated at the rear end of the piston must travel.

US Patent Number 5,135,511 describes an adaptor for traditional syringes that makes possible only sucking with only one hand. The adaptor is made up of two components in addition to the two components which make up a traditional syringe, thus complicating assembly of the same. Use of this adaptor may give rise to a greater number of failures during its use, due to the greater number of components.

The syringe described in US Patent Number 5,582,595, unlike the above-mentioned syringes or adaptors, allows a single hand to be used both for sucking and for injection. The main problem of this syringe lies in assembling it, which may give rise to failures in its use. The syringe is made up of two bodies, the cylinder provided with two rods as an extension towards its rear part with a coupling surface and the piston with two supporting surfaces at its end. The problem stems from the fact that in order to assemble the syringe, the piston has to be introduced in the rear part of the cylinder, which means that the existing surface at the rear end of the cylinder must be capable of pivoting with respect to one of the rods. This makes the manufacturing process of the product and its assembly more expensive. It also means that the person using it must handle it more delicately and carefully because the pivoting surface may become detached during sucking. In this syringe, the piston is similar to that of traditional syringes, being the only element which moves with respect to the cylinder, the rods remaining fixed since they are joined to the cylinder.

The greatest drawback of this model of syringe during use is that it is difficult to control the needle, since the forces required to use it are exerted at a greater distance from the needle than in traditional syringes.

By means of the syringe that is the object of the present invention, the drawbacks involved in the foregoing syringes are resolved, since it enables sucking and expulsion to be carried out with great simplicity by flexing the fingers naturally with comfortable, stable and safe force, controlling the movement and the needle of the syringe at all times. None of said documents allows a syringe to be manufactured that can be used with only one hand and that is provided with two independent bodies which can easily and quickly be assembled, as simple as the syringe described below.

### DESCRIPTION

The present invention, syringe and method for absorbing and expelling a liquid by means of the same, is provided with a particular structure which allows it to be used with only one hand and also allows stable handling, since the two operations of absorption and extraction of a liquid can be carried out with the same action of pressure or flexing of the fingers. Traditional syringes have to be used with two hands during sucking because if they are used with only one hand, the user of the syringe or the patient may be pricked or wounded.

To use a syringe with only one hand, different structural modifications have been made to traditional syringes, but simplicity of use and manufacture have been maintained.

The syringe is provided with two bodies or elements, one cylindrical with the inlet and/or outlet orifice of the liquid at one end, two side longitudinal grooves which are open at the opposite end and a central supporting surface that has a rear part or second supporting surface and another front part or third supporting surface, with a central cavity situated transversely to the cylinder.

The second body is made up of a rod with a piston at one end and two connection links with two extended members, or pushers, at the opposite end. On the side that extends towards the free end (the end to be introduced into the cylinder), the extended members or pushers end in two supporting surfaces (fourth supporting surfaces) which are transverse to the same, while at the opposite end both extended members or pushers are joined by another supporting surface (first supporting surface).

The thickness of the extended members will be greater or smaller according to the diameter of the syringe. In the case of syringes in which the cylinder has a small diameter, the thickness of said extended members at the end that covers the rod will be greater in order to give greater stability to the movement of the rod and also to allow the rear aperture to be widened where the finger is to be introduced. These extended members in large-diameter syringes will have a smaller or similar thickness to that of the extended members.

It is also possible to provide the syringes with two tabs joined to the ends of the extended members to prevent foreign bodies from entering the grooves along which the connection links move.

To use the syringe, it is necessary to place the fingers of one hand in a particular way, as described below. The syringe has, as in the case of traditional syringes, two end positions, one for sucking and the other for expelling liquid. In the first position, ready for sucking and with the piston close to or in contact with the cylinder's orifice, the fingers, any fingers except the thumb, are positioned on the fourth supporting surfaces, and the thumb rests on the second supporting surface in such a way that it is positioned in the window delimited between the second supporting surface and the first supporting surface. Once the fingers are positioned, no more is needed than to make a pressing or flexing action with the fingers, thus producing two contrary forces parallel to the movement of the piston, causing the relative movement of both bodies and, therefore, the sucking up of the liquid.

To expel the liquid inside the cylinder of the syringe, which is found after sucking in its second position, the position of the fingers must be changed, without needing to let go of the syringe nor to help oneself with the other hand. To do this, the thumb is placed on the first supporting surface and two other fingers rest upon the third supporting surface. With the fingers in these positions, the fingers carry out another pressing or flexing action, thus producing two contrary forces parallel to the movement of the piston, causing the relative movement of both bodies and, therefore, the expulsion or injection of the liquid.

Due to the above-mentioned structure, it is not necessary to turn the hand, the wrist or the syringe in order to use the syringe. It is enough to use three fingers of one hand to change, consecutively, the action of absorption and expulsion of liquid in a safe and stable way.

Because handling of the syringe is simple, it is also easy to learn, requiring no training of any kind. By means of the syringe, two functions can be carried out quickly and safely, preventing the user injuring him or herself due to bad handling and suppressing uncontrolled movements.

The movements that govern absorption and expulsion will always be stable, safe and completely controlled, as the direction of the force exerted by the thumb in the two actions coincides with the axis of the movement of the rod and piston inside the cylinder.

Due to its design, it is not necessary to let go of the syringe, nor to help oneself with the other hand, nor to turn the hand or the syringe in order to change the position of the two fingers when changing from the sucking position to the expulsion position or vice versa.

The safety, stability and complete control during sucking and expulsion is also due to the fact that the pressing or flexing action of the fingers is the same in the two actions, with only the position of the fingers changing, thus contributing to the ease with which the syringe is handled and learnt.

The use of only one hand in a stable and controlled way prevents the user from sustaining involuntary pricks and cuts when sucking up a liquid with the help of a needle, thus avoiding the risk of infection.

In the case of sucking up blood or introducing a liquid into a vein, this syringe provides the advantage that, as only one hand is required, the fingers of the other hand may be used to keep the vein constantly located.

The structure of the syringe that is the object of the present invention is applicable to syringes with one or several uses and may be used in administering medicinal injections, blood extractions, biopsy piercing, etc.

### DESCRIPTION OF THE DRAWINGS

To aid understanding of the syringe and the method for absorbing and for expelling a liquid by means of the same, seven drawings are attached to the present patent application, whose purpose is to promote a better understanding of the foundations on which the invention in question is based and a better understanding of the description of a preferred embodiment, taking into account that the drawings are illustrative and non-restrictive.
Figure 1 shows an elevation view of the syringe that is the object of the present patent application prepared to absorb a liquid.

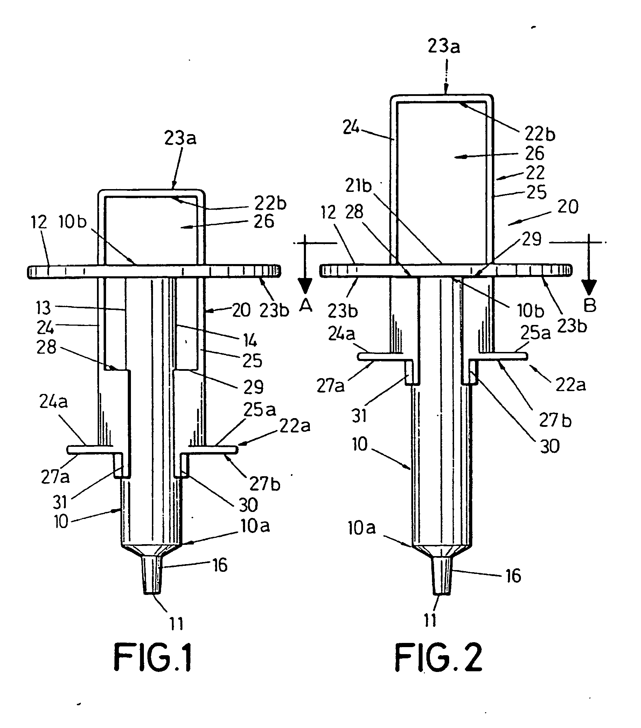

Figure 2 shows an elevation view of the syringe ready to expel the liquid previously absorbed.

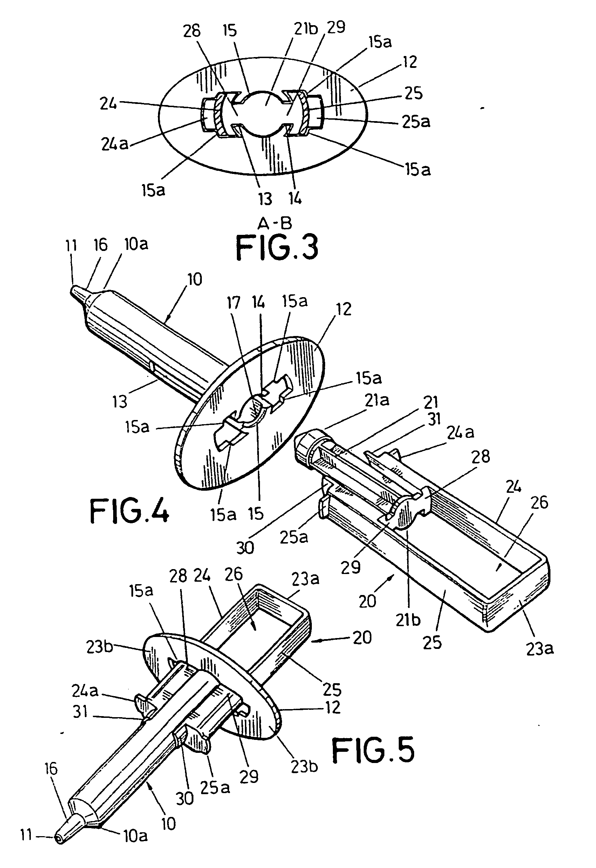

Figure 3 shows an upper plan view of the lower body or first body.
Figure 4 shows an exploded perspective view of the two parts of the syringe.
Figure 5 shows a perspective view of the syringe.
Figure 6 shows an elevation view of the syringe being used during absorption of a liquid, in the first position, in which one may observe the position of the fingers of the hand of the user of the syringe.
Figure 7 shows the elevation view of the syringe being used during expulsion of a liquid, in the second position, in which one may observe the position of the fingers of the hand of the user of the syringe.

### DESCRIPTION OF A PREFERRED EMBODIMENT

The present invention, syringe and method of use, has a novel arrangement of the different elements of a traditional syringe. It also includes new elements which allow the two basic actions of a syringe, sucking or absorption and expulsion, to be carried out by the same pressing or flexing force of the fingers with only one of the user's hands, while maintaining stability during use of the syringe and therefore making said use easy and safe.

The syringe has two bodies or independent basic structures which are preferably moulded in a single piece, each with a particular composition. The first body 10 is cylindrical and has a front end 10a in which an inlet and/or outlet orifice 11 for a liquid is situated and a second open end or mouth 10b. At the front end of the syringe 10a, in addition to the inlet and/or outlet orifice 11, it has means 16 for coupling a hypodermic needle to said front end 10a.

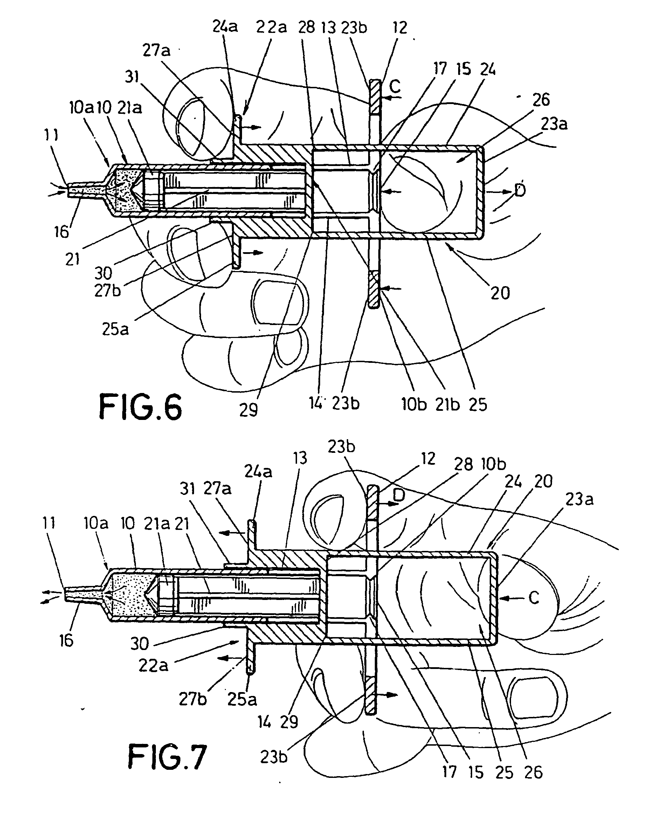

The second body 20 is made up of a first part or rod 21, which is inserted into the first body or cylinder 10 through the mouth 10b of said body 10. The rod 21 has a piston 21a at its end close to the orifice of the syringe 11 and a check 21b at the opposite end. The second body 20 can move between two positions, a first position (figure 6) in which the piston 21a is in a position close to the front end 10a of the cylinder 10; and a second position (figure 7) in which the piston 21a is in a position away from the front end 10a of the cylinder 10. In this way, to absorb a liquid through the orifice 11, the second body 20 moves from the first position to the second position, and to expel a liquid contained in the syringe through the orifice 11, the second body 20 moves from the second position to said first position.

The syringe has four basic supporting surfaces for its operation distributed between its two bodies 10, 20.

The second body 20 is divided into two parts, a rod 21, already described, situated at one end of said body 20 and a second part or pusher 22. Said pusher 22 has two extended members 24, 25 which are joined at their rear part 22b by the first supporting surface 23a, upon which pressure will be exerted in a first axial direction C to move the second body 20 from said second position towards said first position.

The cylinder 10 includes, at its mouth 10b, a supporting element or central supporting surface 12 which projects transversely with respect to the cylinder and forms an integral part of the cylinder 10. This supporting element 12 constitutes a second supporting surface 12a made up of the rear side of the supporting element 12 and a third supporting surface 23b made up of the frontal area of said supporting element 12. On this third supporting element 23b pressure is exerted in an axial direction D, opposite to the first axial direction C, to move the second body 20 from the second position towards the first position.

The pusher 22 of the second body 20 has in its front end 22a two fourth supporting surfaces 27a, 27b, made up of two side flanges 24a, 25a, each of which is situated at the free end of one of the extended members 24, 25, upon which pressure may be exerted in a second axial direction, opposite to the first axial direction, to move the second body 20 from the first position towards the second position.

The thickness of the extended members 24, 25 will be greater or smaller according to the diameter of the syringe. In the case of syringes in which the cylinder 10 has a small diameter, the thickness of said extended members 24, 25 at the front end 22a that covers the rod will be greater in order to give greater stability to the movement of the body 21 and also to allow the aperture 26 to be widened in order to introduce the finger. These extended members 24, 25, at their front end 22a, in large-diameter syringes will have a smaller or similar thickness to that of the extended members.

It is also possible to provide the syringes with two tongues 30, 31 joined to the ends of the extended members 24, 25 to prevent foreign bodies from entering the grooves 13, 14 along which the connection links 28, 29 move.

In order to be able to exert the force in the same direction as that of the movement of the piston during sucking, the syringe has a window or aperture 26 in the second body 20. Said aperture 26 is large enough to allow the user's thumb to be introduced into the same when the second body 20 is in the first position. Said aperture 26 in this position, prior to sucking up a liquid, is delimited at the mouth 10b of the cylinder 10 by the side extended members 24, 25, which are joined to the rod 21 of the second body 20 by the connection links 28, 29 and joined in turn at the rear end 22b of the second body 20 by the first supporting surface 23a. During sucking, said aperture or window 26 increases in size until it reaches the maximum in the second position. Said aperture 26 is then delimited by a check 21b in the rod 21, by the extended members 24, 25 and by the first supporting surface 23a which joins said extended members 24, 25 at the rear end 22b.

To allow the second body 20 to move in relation to the first body 10, the latter includes at its mouth 10b at least two longitudinal grooves 13, 14 which extend from said mouth 10b to the front end 10a of the cylinder. In these longitudinal grooves 13, 14 the connection links 28, 29 are housed which join the extended members 24, 25 of the pusher 22 and the check 21b of the rod 21. The grooves 13, 14 extend from the mouth 10b of the cylinder 10 to the position that the connection links 28, 29 occupy when the piston 21a of the rod 21 is situated in the first position, that is, in contact with the orifice 11.

When the second body 20 is in the first position, that is prepared to suck or absorb a liquid, the check 21b of the rod 21 is situated at a distance from the mouth 10b of the cylinder 10 which corresponds to the extent of the movement of the second body 20 between said first position and said second position. Once suction is completed and prior to expulsion of the liquid, that is, the second body 20 is in the second position, the check 21b of the rod 21 is situated at the same level as the mouth 10b of the cylinder 10.

The above-mentioned supporting element 12, which serves as a central supporting surface, has an open orifice 15 shaped in a way which allows the entry, in an axial direction, of the rod 21, of the connection links 28, 29, of the fourth supporting surfaces 27a, 27b and part of the extended members 24, 25. Said open orifice 15 has side supporting edges 15a which serve to hold the extended members 24, 25 in place.

To change from the second position to the first position, that is, prior to the expulsion of the liquid contained in the syringe, it is necessary to position the fingers on the third supporting surface 23b. Therefore the distance between this supporting surface 23b and the front end 22a of the pusher 22 is such that the user may place one finger on each side of the syringe.

The syringe has a special application in the extraction of blood, so that when it is necessary to pierce a vein, we may keep the vein located with a finger of one hand while with the other hand we may use the syringe to suck or extract blood as well as to expel or introduce medicine.

To use the syringe for absorbing a liquid it is necessary to place the orifice 11 of the first body 10 in fluid contact with the liquid and situate the rod 21 in the first position. Once this has been done, the fingers of the hand must be situated on the different surfaces of the syringe so that the thumb is placed on the aperture 26 delimited by the check 21b of the rod 21, by the extended members 24, 25 and by the first supporting surface 23a at the rear end 22b of the pusher 22; simultaneously, two fingers of the same hand must be placed on the fourth supporting surfaces 27a, 27b. To proceed to suck the liquid using the syringe, pressure must be exerted by the thumb on the second supporting surface 12a in a first axial direction C; and simultaneously pressure must be exerted by the two fingers of the same hand on the fourth supporting surfaces 27a, 27b, in a second axial direction D opposite to the first axial direction C, in order to move the second body 22 from the first position to the second position.

In this action, the check 21b of the rod 21 helps in not loading the syringe to more than its maximum capacity, since at the end of the sucking action, the check 21b comes into contact with the thumb introduced into the aperture 26 and situated upon the second supporting surface 12a.

In addition to this measure which prevents extraction of the rod 21 from the cylinder 10, in the inner surface of the cylinder there is a ring 17 in relief, close to the mouth 10b of the cylinder 10, whose purpose is to make it difficult to extract said rod 21 from the cylinder 10.

To expel the liquid previously introduced into the syringe, it is necessary to position the thumb on the first supporting surface 23a and two fingers of the same hand on the third supporting surface 23b. Once the fingers are placed on the syringe and the orifice 11 is situated over a receptacle or test tube for receiving the contents of the syringe, the thumb exerts pressure in the first axial direction and the fingers exert pressure in the second axial direction opposite the first axial direction in order to move the second body 22 from the second position to the first position.

To carry out the foregoing actions in a stable, safe and simple way with only one hand, the syringe must have a particular and characteristic structure that allows the pressure exerted by the fingers of the hand on the syringe to be carried out in such a way that the direction of the force applied is the same as the direction of the piston movement, whether in one direction or another, according to whether the sucking or expulsion movement is being carried out.

Within the bounds of its essential nature, the invention may be carried into practice in other embodiments differing only in detail from that indicated here merely by way of example. Thus it may be embodied in any shape and size, with the most appropriate means and materials and the most convenient accessories, and the component elements may be replaced by others that are technically equivalent, as long as they are included within the scope of the claims.

## Claims

1. A syringe, made up of:
- a first body (10) with the shape of a cylinder which has a front end (10a) in which an inlet and/or outlet orifice (11) for liquid is situated and a second open end or mouth (10b)
- a second body (20) made up of a first part or rod (21) which has a piston (21a) at a front end, said rod (21) of the second body (20) being inserted in the cylinder (10) through the mouth (10b) of said cylinder (10); said second body (20) can be moved between a first position in which the piston (21a) of the rod (21) is in a position close to the front end (10a) of the cylinder (10), and a second position in which said piston (21a) of the rod (21) is in a position away from the front end (10a) of the cylinder (10), so that to absorb a liquid through the orifice (11) the second body (20) is movable from said first position to said second position and to expel a liquid through the orifice (11) the second body (20) is movable from said second position to said first position;
said second body (20) has a second part or pusher (22) which has a front end (22a) and a rear end (22b); said rear end (22b) of the pusher (22) constitutes a first supporting surface (23a) upon which pressure can be exerted in a first axial direction to move the second body (20) from the second position to the first position;
the cylinder (10) includes in its check (10b) a central supporting surface (12) which projects transversely with respect to the cylinder and forms an integral part of the cylinder (10); said central supporting surface (12) constitutes on its rear surface a second supporting surface and on its front surface a third supporting surface (23b) upon which pressure can be exerted in a second axial direction opposite to the first axial direction, to move the second body (20) from the second position to the first position;
**characterised in that**
the pusher (22) of the second body (20) includes two extended members (24, 25) which are joined to the rod (21) of the second body (20) by the connection links (28, 29) and which are joined at the rear end (22b) of the pusher by the first supporting surface (23a), thus establishing an aperture (26) delimited by the check (21b) of the rod (21) by the central supporting surface (12), by the extended members (24, 25) and by the first supporting surface (23a);
**in that**
the pusher (22) of the second body (20) forms, at its front end (22a), a fourth supporting surface (27a, 27b) upon which pressure can be exerted in a second axial direction, opposite to the first axial direction, to move the second body (20) from the first position to the second position;
**in that**
the cylinder (10) includes, in its mouth (10b), at least two longitudinal grooves (13, 14) which extend from said mouth (10b) to the front end (10a) of the cylinder (10) to the place occupied by the check (21b) of the rod in the first position;
and **in that**
the extended members (24, 25) of the pusher (22) of the second body (20) are joined to the check (21b) of the rod (21) of said second body (20) by means of the connection links (28, 29) which are housed in said longitudinal grooves (13, 14).

2. A syringe in accordance with claim 1, **characterised in that** when the second body (20) is in the first position, the aperture (26) is large enough to allow the user's thumb to be introduced into said aperture (26); the aperture (26) is delimited in this position by the mouth (10b) of the first body (10), by the central supporting surface (12), by the extended side members (24, 25) and by the first supporting surface (23a) which joins said extended members (24, 25) at their rear end (22b).

3. A syringe in accordance with any of the foregoing claims, **characterised in that** when the second body (20) is in the first position, the check (21b) of the rod (21) is situated at a distance from the mouth (10b) of the cylinder (10) that corresponds to the extent of the movement of the second body (20) between said first position and the second position.

4. A syringe in accordance with any of the foregoing claims, **characterised in that** when the second body (20) is in the second position, the check (21b) of the rod (21) of the second body (20) is situated at the same level as the mouth (10b) of the cylinder (10).

5. A syringe in accordance with any of the foregoing claims, **characterised in that** the supporting element (12) has an open orifice (15) shaped in a way which allows the entry, in an axial direction, of the rod (21) of the second body (20), the connection links (28, 29), the fourth supporting surfaces (27a, 27b) and part of the extended members (24, 25).

6. A syringe in accordance with any of the foregoing claims, **characterised in that** the open orifice (15) of the supporting element (12) has side supporting edges (15a) which serve to hold the extended members (24, 25) in place.

7. A syringe in accordance with any of the foregoing claims, **characterised in that** the grooves (13, 14) extend from the mouth (10b) of the cylinder (10) to the position that the connection links (28, 29) occupy when the piston (21a) of the rod (21) is situated in contact with the orifice (11).

8. A syringe in accordance with claim 7, **characterised in that** it has two tabs (30, 31) joined to the end (22a) of the extended members (24, 25) to prevent any foreign bodies from entering the grooves (13, 14) and to prevent the connection links (28, 29) from moving along them.

9. A syringe in accordance with any of the foregoing claims, **characterised in that** when the second body (20) is in its second position, the distance between the third supporting surface (23b) and the front end (22a) of the pusher (22) of the second body (20) is such that a user can place a finger on each side of the syringe, between said front end (22a) of the pusher (22) of the second body (20) and said third supporting surface (23b).

10. A syringe in accordance with any of the foregoing claims, **characterised in that** the fourth supporting surfaces (27a, 27b) are made up of two side flanges (24a, 25a), each one of which is situated at the free end of one of the extended members (24, 25).

11. A syringe in accordance with any of the foregoing claims, **characterised in that** it is used with only one hand.

12. A syringe in accordance with any of the foregoing claims, **characterised in that** at the front end (10a) of the cylinder (10) means (16) are provided for coupling a hypodermic needle to said front end (10a) of the cylinder (10) .

13. A syringe in accordance with any of the foregoing claims, **characterised in that** the first body (10) is a body moulded in one piece.

14. A syringe in accordance with any of the foregoing claims, **characterised in that** the second body (20) is a body moulded in one piece.

15. A syringe in accordance with the first claim, **characterised in that** the thickness of the extended members (24, 25) at the front end of the pusher (22a) is greater or smaller according to the diameter of the cylinder (10) of the syringe.

16. A syringe in accordance with claim 15, **characterised in that** for syringes with a small cylinder diameter (10), the thickness of the extended members (24, 25) at the front end of the pusher (22a) will be greater than that of the extended members (24, 25) to allow the thumb to be introduced into the aperture (26) which is delimited in width by the separation between the extended members (24, 25) .

17. A syringe in accordance with claim 15, **characterised in that** for syringes with a large cylinder diameter (10), the thickness of the extended members (24, 25) at the front end of the pusher (22a) will be smaller or similar to that of the extended members (24, 25) to allow the thumb to be introduced into the aperture (26) which is delimited in width by the separation between the extended members (24, 25).

18. A syringe in accordance with the foregoing claims, **characterised in that** it has in the inner surface of the cylinder (10) at least one ring (17) in relief, close to the mouth (10b) of the cylinder (10), to prevent the rod (21) from being extracted from the cylinder (10).

## Patentansprüche

1. Spritze, bestehend aus:
- einem ersten Körper (10), der zylinderförmig ausgebildet ist und ein vorderes Ende (10a), an dem sich eine Eingangs- und/oder Ausgangsöffnung (11) für Flüssigkeiten befindet, sowie eine zweite Öffnung oder Mündung (10b) aufweist;
- einem zweiten Körper (20), bestehend aus einem ersten Teil oder Stange (21), die an einem vorderen Ende einen Kolben (21a) aufweist, wobei die genannte Stange (21) des genannten zweiten Körpers (20) in den Zylinder (10) über die Mündung (10b) dieses Zylinders (10) eingebracht ist; wobei der genannte zweite Körper (20) zwischen einer ersten Position, in der Kolben (21a) der Stange (21) nahe bei dem vorderen Ende (10a) des Zylinders liegt (10), und einer zweiten Position, in der der genannte Kolben (21a) der Stange (21) in einer Position, beweglich ist, wobei der zweite Körper (20) zwischen der genannten ersten Position bis zur genannten zweiten Position beweglich ist, um über die Öffnung (11) eine Flüssigkeit aufzunehmen, und wobei der zweite Körper (20) von der genannten zweiten Position bis zur genannten ersten Position beweglich ist, um eine Flüssigkeit über die Öffnung (11) auszustoßen;
wobei der genannte zweite Körper (20) ein zweites Teil bzw. einen Stößel (22) aufweist, der ein vorderes Ende (22a) und ein hinteres Ende (22b) hat; wobei das genannte hintere Ende (22b) des Stößels (22) eine erste Auflagefläche (23a) bildet, auf die in eine erste axiale Richtung Druck ausgeübt werden kann, um den zweiten Körper (20) von der zweiten Position in die erste Position zu bewegen;
wobei der Zylinder (10) an seinem Anschlag (10b) eine zentrale Auflagefläche (12) aufweist, die sich hinsichtlich des Zylinders in die Querrichtung erstreckt und die einstückig mit dem Zylinder (10) verbunden ist; wobei diese zentrale Auflagefläche (12) auf der hinteren Fläche eine zweite Auflagefläche und auf der vorderen Fläche eine dritte Auflagefläche (23b) bildet, auf die in eine zweite axiale Richtung, die der ersten axialen Richtung entgegengesetzt ist, Druck ausgeübt werden kann, um den zweiten Körper (20) von der zweiten Position in die erste Position zu bewegen;
**dadurch gekennzeichnet, dass**
der Stößel (22) des zweiten Körpers (20) zwei verlängerte Elemente (24, 25) aufweist, die über Anschlussverbindungen (28, 29) mit der Stange (21) des zweiten Körpers (10) verbunden sind und die über die erste Auflagefläche (23a) mit dem hinteren Ende (22b) des Stößels verbunden sind, **dadurch** dass eine von dem Anschlag (21b) der Stange (21), der zentralen Auflagefläche (12), den verlängerten Elementen (24, 25) und der ersten Auflagefläche (23a) begrenzte Öffnung (26) festgelegt ist;
dass
der Stößel (22) des zweiten Körpers (20) am vorderen Ende (22a) eine vierte Auflagefläche (27a, 27b) bildet, auf die in eine zweite axiale Richtung, die der ersten axialen Richtung entgegengesetzt ist, Druck ausgeübt werden kann, um den zweiten Körper (20) von der ersten Position in die zweite Position zu bewegen;
dass
der Zylinder (10) an seiner Mündung (10b) zumindest zwei sich in Längsrichtung erstreckende Schlitze (13, 14) aufweist, die sich von der genannten Mündung (10b) bis zum vorderen Ende (10a) des Zylinders (10), bis zum Ort, an dem sich der Anschlag (21b) der Stange in der ersten Position befindet, erstrecken;
und dass
die verlängerten Elemente (24, 25) des Stößels (22) des zweiten Körpers (20) über die, in den sich in Längsrichtung erstreckenden Schlitzen (13, 14) aufgenommenen Anschlussverbindungen (28, 29) mit dem Anschlag (21b) der Stange (21) des genannten zweiten Körpers (20) verbunden sind.

2. Spritze nach Anspruch 1, **dadurch gekennzeichnet, dass**, wenn der zweite Körper (20) sich in der ersten Position befindet, die Öffnung (26) ausreichend groß ist, um zu ermöglichen, dass der Daumen des Benutzers in die genannte Öffnung (26) eingeführt werden kann; wobei die Öffnung (26) in dieser Position von der Mündung (10b) des ersten Körpers (10), der zentralen Auflagefläche (12), den verlängerten seitlichen Elementen (24, 25) und der ersten Auflagefläche (23a), die die beiden genannten verlängerten Elemente (24, 25) an ihrem hinteren Ende (22b) verbindet, begrenzt ist.

3. Spritze nach einem der vorhergehenden Ansprüche, **dadurch gekennzeichnet, dass**, wenn sich der zweite Körper (20) in der ersten Position befindet, der Anschlag (21b) des Stabes (21) sich in einer Entfernung von der Mündung (10b) des Zylinders (10) befindet, die dem Bewegungshub des zweiten Körpers (20) zwischen der genannten ersten Position und der zweiten Position entspricht.

4. Spritze nach einem der vorhergehenden Ansprüche, **dadurch gekennzeichnet, dass**, wenn sich der zweite Körper (20) in der zweiten Position befindet, der Anschlag (21b) des Stabes (21) des zweiten Körpers (20) sich auf der gleichen Höhe wie die Mündung (10b) des Zylinders (10) befindet.

5. Spritze nach einem der vorhergehenden Ansprüche, **dadurch gekennzeichnet, dass** die Auflagefläche (12) eine offene Öffnung (15) aufweist, die so ausgebildet ist, dass sie den Eintritt in axialer Richtung des Stabes (21) des zweiten Körpers (20), der Anschlussverbindungen (28, 29), der vierten Auflageflächen (27a, 27b) und eines Teils der verlängerten Elemente (24, 25) ermöglicht.

6. Spritze nach einem der vorhergehenden Ansprüche, **dadurch gekennzeichnet, dass** die offene Öffnung (15) der Auflagefläche (12) seitliche Auflageränder (15a) aufweist, die dazu dienen, die verlängerten Elemente (24, 25) an dem ihnen zustehenden Platz zu halten.

7. Spritze nach einem der vorhergehenden Ansprüche, **dadurch gekennzeichnet, dass** die Schlitze (13, 14) sich von der Mündung (10b) des Zylinders (10) bis zu der Position erstrecken, die die Anschlussverbindungen (28, 29) einnehmen, wenn sich der Kolben (21a) des Stabes (21) in Kontakt mit der Öffnung (11) befindet.

8. Spritze nach Anspruch 7, **dadurch gekennzeichnet, dass** sie zwei mit dem Ende (22a) der verlängerten Elemente (24, 25) verbundene Zungen (30, 31) aufweist, um zu vermeiden, dass ein Fremdkörper zwischen die Schlitze (13, 14) eintreten kann und um zu vermeiden, dass die Anschlussverbindungen (28, 29) sich entlang dieser Schlitze bewegen.

9. Spritze nach einem der vorhergehenden Ansprüche, **dadurch gekennzeichnet, dass**, wenn sich der zweite Körper (20) in der zweiten Position befindet, der Abstand zwischen der dritten Auflagefläche (23b) und dem vorderen Ende (22a) des Stößels (22) des zweiten Körpers so bemessen ist, dass ein Benutzer einen Finger auf jede Seite der Spritze, zwischen dem genannten vorderen Ende (22a) des Stößels (22) des zweiten Körpers (20) und der genannten dritten Auflagefläche (23b) anlegen kann.

10. Spritze nach einem der vorhergehenden Ansprüche, **dadurch gekennzeichnet, dass** die vierten Auflageflächen (27a, 27b) aus zwei seitlichen vorspringenden Rändern (24a, 25a) bestehen, wobei jeder dieser Ränder sich am freien Ende eines der verlängerten Elemente (24, 25) befindet.

11. Spritze nach einem der vorhergehenden Ansprüche, **dadurch gekennzeichnet, dass** diese mit nur einer Hand bedient wird.

12. Spritze nach einem der vorhergehenden Ansprüche, **dadurch gekennzeichnet, dass** am vorderen Ende (10a) des Zylinders (10) Mittel (16) bereitstehen, um eine subkutane Nadel an dem genannten vorderen Ende (10a) des Zylinders (10) anzubringen.

13. Spritze nach einem der vorhergehenden Ansprüche, **dadurch gekennzeichnet, dass** der erste Körper (20) ein einstückiges Formteil ist.

14. Spritze nach einem der vorhergehenden Ansprüche, **dadurch gekennzeichnet, dass** der zweite Körper (20) ein einstückiges Formteil ist.

15. Spritze nach Anspruch 1, **dadurch gekennzeichnet, dass** die Dicke der verlängerten Elemente (24, 25) am vorderen Ende des Stößels (22a) größer oder kleiner sein kann, in Abhängigkeit des Durchmessers des Zylinders (10) der Spritze.

16. Spritze nach Anspruch 15, **dadurch gekennzeichnet, dass** für die Spritzen mit einem kleinen Durchmesser (10) des Zylinders, die Dicke der verlängerten Elemente (24, 25) am vorderen Ende des Stößels (22a) größer ist als die Dicke der verlängerten Elemente (24, 25), um zu ermöglichen, dass der Daumen in die Öffnung (26) eingeführt werden kann, deren Breite durch den Abstand zwischen den verlängerten Elementen (24, 25) begrenzt ist.

17. Spritze nach Anspruch 15, **dadurch gekennzeichnet, dass** für die Spritzen mit einem großen Durchmesser des Zylinders (10), die Dicke der verlängerten Elemente (24, 25) am vorderen Ende des Stößels (22a) kleiner oder ähnlich ist wie die Dicke der verlängerten Elemente (24, 25), um zu ermöglichen, dass der Daumen in die Öffnung (26) eingeführt werden kann, deren Breite durch den Abstand zwischen den verlängerten Elementen (24, 25) begrenzt ist.

18. Spritze nach den vorhergehenden Ansprüchen, **dadurch gekennzeichnet, dass** sie an der Innenfläche des Zylinders (10) zumindest einen erhöhten Ring (17) aufweist, der nahe bei der Mündung (10b) des Zylinders (10) liegt, um zu vermeiden, dass der Stab (21) aus dem Zylinder (10) herausgezogen wird.

## Revendications

1. Seringue, composée de:
- un premier corps (10) sous forme d'un cylindre ayant une extrémité (10a) avant dans laquelle est situé un orifice (11) d'entrée et/ou de sortie pour des liquides et une deuxième ouverture ou embouchure (10b);
- un deuxième corps (20) composé d'une première partie ou tringle (21) qui a un piston (21a) à une extrémité avant, ladite tringle (21) dudit deuxième corps (20) étant insérée dans le cylindre (10) à travers l'embouchure (10b) dudit cylindre (10); ledit deuxième corps (20) pouvant se déplacer entre une première position dans laquelle le piston (21a) de la tringle (21) est dans une position proche de l'extrémité (10a) avant du cylindre (10), et une deuxième position dans laquelle ledit piston (21a) de la tringle (21) est dans une position éloignée de l'extrémité (10a) avant du cylindre (10), de telle sorte que pour absorber un liquide à travers l'orifice (11), le deuxième corps (20) peut se déplacer depuis cette première position jusqu'à ladite deuxième position, et pour expulser un liquide à travers l'orifice (11), le deuxième corps (20) peut se déplacer depuis ladite deuxième position jusqu'à ladite première position;
ledit deuxième corps (20) ayant une deuxième partie ou pousseur (22) qui a une extrémité (22a) avant et une extrémité (22b) arrière; ladite extrémité (22b) arrière du pousseur (22) constituant une première surface (23a) de support sur laquelle on peut exercer de la pression dans une première direction axiale pour déplacer le deuxième corps (20) depuis la deuxième position jusqu'à la première position;
le cylindre (10) incluant dans sa butée (10b) une surface (12) de support centrale qui se projette transversalement par rapport au cylindre et forme une partie intégrale du cylindre (10); ladite surface (12) de support centrale constituant sur sa surface arrière une deuxième surface de support et sur sa surface avant une troisième surface (23b) de support sur laquelle on peut exercer de la pression dans une deuxième direction axiale opposée à la première direction axiale, pour déplacer le deuxième corps (20) depuis la deuxième position jusqu'à la première position;
**caractérisée en ce que**
le pousseur (22) du deuxième corps (20) inclut deux éléments (24, 25) étendus qui sont unis à la tringle (21) du deuxième corps (20) au moyen de raccords (28, 29) de connexion et qui sont unis à l'extrémité (22b)arrière du pousseur au moyen de la première surface (23a) de support, en disposant ainsi une ouverture (26) délimitée par la butée (21b) de la tringle (21) au moyen de la surface (12) de support centrale, au moyen des éléments (24, 25) étendus et au moyen de la première surface (23a) de support;
**en ce que**
le pousseur (22) du deuxième corps (20) forme, à son extrémité (22a) avant, une quatrième surface (27a, 27b) de support sur laquelle on peut exercer de la pression dans une deuxième direction axiale, opposée à la première direction axiale, pour déplacer le deuxième corps (20) depuis la première position jusqu'à la deuxième position;
**en ce que**
le cylindre (10) inclut, dans son embouchure (10b), au moins deux fentes (13, 14) longitudinales qui s'étendent depuis ladite embouchure (10b) jusqu'à l'extrémité (10a) avant du cylindre (10) jusqu'au lieu occupé par la butée (21b) de la tringle dans la première position;
et **en ce que**
les éléments (24, 25) étendus du pousseur (22) du deuxième corps (20) sont unis à la butée (21b) de la tringle (21) dudit deuxième corps (20) au moyen des raccords (28, 29) de connexion qui sont logées dans lesdites fentes (13, 14) longitudinales.

2. Seringue selon la revendication 1, **caractérisée en ce que** lorsque le deuxième corps (20) est dans la première position, l'ouverture (26) est suffisamment grande pour permettre que le pouce de l'utilisateur soit introduit dans ladite ouverture (26); l'ouverture (26) étant délimitée à cette position par l'embouchure (10b) du premier corps (10), par la surface (12) de support centrale, par les éléments (24, 25) latéraux étendus et par la première surface (23a) de support qui unit lesdits éléments (24, 25) étendus à son extrémité (22b) arrière.

3. Seringue selon l'une quelconque des revendications antérieures, **caractérisée en ce que** lorsque le deuxième corps (20) est dans la première position, la butée (21b) de la tringle (21) est située à une distance depuis l'embouchure (10b) du cylindre (10) qui corresponde à l'amplitude du mouvement du deuxième corps (20) entre ladite première position et la deuxième position.

4. Seringue selon l'une quelconque des revendications, **caractérisée en ce que** lorsque le deuxième corps (20) est à la deuxième position, la butée (21b) de la tringle (21) du deuxième corps (20) est située au même niveau que l'embouchure (10b) du cylindre (10).

5. Seringue selon l'une quelconque des revendications antérieures, **caractérisée en ce que** l'élément (12) de support a un orifice (15) ouvert conformé d'une façon qui permet l'entrée, dans une direction axiale, de la tringle (21) du deuxième corps (20), les raccords (28, 29) de connexion, les quatrièmes surfaces (27a, 27b) de support et une partie des éléments (24, 25) étendus.

6. Seringue selon l'une quelconque des revendications antérieures, **caractérisée en ce que** l'orifice (15) ouvert de l'élément (12) de support a des bords (15a) de support latéraux qui servent pour retenir les éléments (24, 25) étendus à leur place.

7. Seringue selon l'une quelconque des revendications antérieures, **caractérisée en ce que** les fentes (13, 14) s'étendent depuis l'embouchure (10b) du cylindre (10) jusqu'à la position que occupent les raccords (28, 29) de connexion lorsque le piston (21a) de la tringle (21) est situé en contact avec l'orifice (11).

8. Seringue selon la revendication 7, **caractérisée en ce qu'**elle a deux languettes (30, 31) unies à l'extrémité (22a) des éléments (24, 25) étendus pour éviter que tout corps étranger rentre dans les fentes (13, 14) et pour éviter que les raccords (28, 29) de connexion se déplacent le long de celles-ci.

9. Seringue selon l'une quelconque des revendications antérieures, **caractérisée en ce que** lorsque le deuxième corps (20) est dans sa deuxième position, la distance entre la troisième surface (23b) de support et l'extrémité (22a) avant du pousseur (22) du deuxième corps (20) est telle qu'un utilisateur peut en mettre un doigt de chaque côté de la seringue, entre ladite extrémité avant (22a) du pousseur (22) du deuxième corps (20) et ladite troisième surface (23b) de support.

10. Seringue selon l'une quelconque des revendications antérieures, **caractérisée en ce que** les quatrièmes surfaces (27a, 27b) de support sont composées de deux rebords (24a, 25a) latéraux, chacun desquels est situé à l'extrémité libre d'un des éléments (24, 25) étendus.

11. Seringue selon l'une quelconque des revendications antérieures, **caractérisée en ce qu'**elle est utilisée avec une seule main.

12. Seringue selon l'une quelconque des revendications antérieures, **caractérisée en ce qu'**à l'extrémité (10a) avant du cylindre (10) on pourvoit des moyens (16) pour coupler une aiguille hypodermique à ladite extrémité (10a) avant du cylindre (10).

13. Seringue selon l'une quelconque des revendications antérieures, **caractérisée en ce que** le premier corps (10) est un corps moulé monobloc.

14. Seringue selon l'une quelconque des revendications antérieures, **caractérisée en ce que** le deuxième corps (20) est un corps moulé monobloc.

15. Seringue selon la première revendication, **caractérisé en ce que** l'épaisseur des éléments (24, 25) étendus à l'extrémité avant du pousseur (22a) est plus grande ou plus petite selon le diamètre du cylindre (10) de la seringue.

16. Seringue selon la revendication 15, **caractérisée en ce que** pour les seringues ayant un diamètre (10) de cylindre faible, l'épaisseur des éléments (24, 25) étendus à l'extrémité avant du pousseur (22a) sera plus grande que celle des éléments (24, 25) étendus pour permettre l'introduction du pouce dans l'ouverture (26) qui est délimitée en largeur par la séparation entre les éléments (24, 25) étendus.

17. Seringue selon la revendication 15, **caractérisée en ce que** pour les seringues ayant un diamètre (10) de cylindre grand, l'épaisseur des éléments (24, 25) étendus à l'extrémité avant du pousseur (22a) sera plus petite ou similaire à celle des éléments (24, 25) étendus pour permettre l'introduction du pouce dans l'ouverture (26) qui est délimitée en largeur par la séparation entre les éléments (24, 25) étendus.

18. Seringue selon les revendications antérieures, **caractérisée en ce qu'**elle a sur la surface interne du cylindre (10) au moins un anneau (17) en relief, près de l'embouchure (10b) du cylindre (10), pour éviter que la tringle (21) soit extraite du cylindre (10).
